Europäisches Patentamt

**European Patent Office**

**Office européen des brevets**

(11) Numéro de publication: **0 042 395**
**B1**

(12) **FASCICULE DE BREVET EUROPEEN**

(45) Date de publication du fascicule du brevet:
**15.02.84**

(21) Numéro de dépôt: **81900065.4**

(22) Date de dépôt: **18.12.80**

(86) Numéro de dépôt international:
**PCT/FR 80/00185**

(87) Numéro de publication internationale:
**WO 81/01860 (09.07.81 Gazette 81/16)**

(51) Int. Cl.³: **C 12 N 11/12,** C 12 P 19/00,
C 13 K 3/00

(54) **ENZYMES IMMOBILISEES SUR UN SUPPORT SOLIDE.**

(30) Priorité: **21.12.79 FR 7931382**

(43) Date de publication de la demande:
**30.12.81 Bulletin 81/52**

(45) Mention de la délivrance du brevet:
**15.02.84 Bulletin 84/7**

(84) Etats contractants désignés:
**AT CH DE GB LI LU NL**

(56) Documents cités:
**FR - A - 2 247 472**
**US - A - 3 947 352**

**O.R. Zaborsky: "Immobilized Enzymes", published on
1974, by The Chemical Rubber Company Press (Ohio,
US), see page 6, right-hand column, lines 30-44; page 7;
tabl.2, point D**

(73) Titulaire: **BEGHIN-SAY SOCIETE ANONYME,
F-59239 Thumeries (FR)**

(72) Inventeur: **MONSAN, Pierre, 73 rue Alfred de Musset,
F-31077 Toulouse (FR)**

ACTORUM AG

Enzymes immobilisées sur un support solide

*Domaine technique*

La présente invention concerne un procédé de fixation d'une enzyme sur un support solide insoluble dans l'eau. Elle vise également un complexe support-enzyme et un procédé d'utilisation d'un tel complexe.

*Technique antérieure*

Depuis plusieurs années, on cherche à insolubiliser des enzymes ou d'autres agents tels des anticorps, des antigènes ou des acides nucléiques de façon à pouvoir purifier, modifier ou isoler certaines substances avec lesquelles ces catalyseurs forment des complexes ou bien favorisent une réaction spécifique.

Les tentatives visant à obtenir des associations support-enzymes insolubles s'expliquent par le fait que des enzymes sont utilisées dans de nombreuses réactions et qu'il est non seulement intéressant, du point de vue économique, de récupérer une enzyme onéreuse mais que, de plus, les produits issus de la réaction ne soient pas souillés.

Le choix des supports et les méthodes de fixation ou d'association des enzymes ont suscité de nombreux travaux: la littérature scientifique est très abondante et décrit plusieurs supports tels la silice, le verre, des polymères synthériques, des polysaccharides et en particulier la cellulose.

La plupart des publications concernant la cellulose visent une cellulose très pure, alors qu'en général la cellulose est associée à des hémicelluloses et de la lignine.

Ainsi, N. Weliky et H. H. Weethall ont publié en 1965 (Immunochemistry Pergamon Press, vol. 2, pp. 293-322) un article intitulé «The Chemistry and use of cellulose derivatives for the study of biological systems».

La cellulose a la réactivité d'un polyalcool et peut être soumise à des réactions d'oxydation, d'estérification, d'éthérification, d'halogénation, etc.

En particulier, les oxycelluloses sont couplées à des alcools, des amines ou des protéines en formant des esters ou des amides.

On comprend d'ailleurs, eu égard à la nature des réactifs employés pour modifier la cellulose, pourquoi l'on a en général cherché à éliminer la lignine — polymère réticulé dont les nombreux groupes réactifs consomment les corps utilisés pour modifier la cellulose ou bien inhibent les réactions envisagées.

Toutefois, le brevet américain N° 3841969 (A. N. Emery et coll.) décrit une méthode de préparation d'enzymes immobilisées par chélation avec un complexe métallique et des substances comportant des fonctions hydroxyles: cellulose microcristalline, cellulose diéthylaminoéthylée, carboxyméthylcellulose mais aussi sciure de bois, copeaux de bois.

Cependant, outre la difficulté d'utilisation industrielle des chlorures métalliques tels le tétrachlorure de titane ou le chlorure d'étain, on sait que les chélates ne sont pas stables dans des gammes étendues de pH.

Le brevet US N° 3947352 décrit une méthode pour fixer une enzyme sur un support à base de polysaccharide faisant intervenir les étapes suivantes:
a) oxydation des groupements hydroxyles par du métaperiodate de sodium,
b) transformation des groupements aldéhydes formés en base de Schiff au moyen d'une diamine,
c) réduction des bases de Schiff formées,
d) couplage de l'enzyme.

Toutefois, ce procédé ne conduit pas à des supports ayant une activité enzymatique suffisante.

D'autre part, le brevet français N° 2247472 (SNPA) décrit une méthode de fixation d'enzymes sur de la cellulose associée à de la lignine, mais indique, ce qui paraît fort étonnant, que la réaction d'association cellulose-enzyme est favorisée par de la lignine présente à raison de 5 à 25% en poids, et de préférence 10 à 20%.

Des grains de rafles de céréales dont la dimension varie entre 0,3 et 10 mm sont tout d'abord délignifiés en surface puis, après lavage, traités avec du chlorure de thionyle dans la pyridine, enfin mis en contact avec une solution tamponnée d'enzyme.

Il semble évident que la lignine n'a aucune influence favorable sur la réaction permettant d'immobiliser l'enzyme puisqu'il est indispensable de commencer par une étape de délignification superficielle des grains.

*Exposé de l'invention*

La présente invention concerne un procédé d'obtention de dérivés enzymatiques dans lesquels l'enzyme est fixée par une liaison covalente sur un support insoluble dans l'eau, comportant les étapes suivantes:
a) lavage à l'eau d'un support contenant essentiellement de la cellulose et de la lignine,
b) oxydation ménagée de la fraction osidique du support,
c) condensation d'une diamine sur les groupes aldéhydiques du support oxydé,
d) stabilisation de la liaison amine-support par réduction,
caractérisé en ce que
e) on active les groupes aminés par un dialdéhyde, et en ce que
f) on couple ensuite l'enzyme sur le support activé.

D'une façon préférentielle, l'oxydant de l'étape b est le periodate de sodium, la diamine est l'éthylènediamine, le réducteur le borohydrure ou le cyanoborohydrure de sodium, le dialdéhyde, le glutaraldéhyde. L'enzyme peut être notamment l'invertase, la lévane saccharase, la lactase ou la dextranesucrase.

Bien que tout support contenant de la cellulose et de la lignine soit utilisable, des résultats particulièrement intéressants ont été obtenus avec la fraction la plus lignifiée des rafles de céréales, notamment des rafles de maïs.

Les rafles de maïs sont constituées:

1) d'éléments tendres appelés «Feeds», contenant en majeure partie de la cellulose et des protéines, destinés à l'alimentation du bétail,
2) d'éléments durs — appelés Grits — contenant une grande proportion de lignine (supérieure à 25%), de la cellulose et des xylanes.

Ces éléments sont en général broyés et utilisés en raison de leur dureté comme charges ou comme abrasifs. Les particules pseudo-sphériques obtenues ont des dimensions pouvant varier entre quelque dizaines de microns et quelques millimètres.

L'invention vise également un dérivé enzymatique dans lequel l'enzyme est liée de façon covalente au support insoluble dans l'eau contenant essentiellement de la cellulose et de la lignine, caractérisé en ce que la liaison entre l'enzyme et le support est assurée par l'enchaînement chimique suivant: $-NH-R-NH=R1=$, R étant le reste d'une diamine, R1 étant le reste d'un dialdéhyde, l'enzyme étant l'invertase, la lévane saccharase, la lactase ou la dextrane.

Un autre objet de l'invention concerne un procédé de traitement de jus sucrés à l'aide d'enzymes fixées par exemple sur la partie des rafles de maïs dont la teneur en lignine est supérieure à 25%.

En particulier, un jus concentré de saccharose est traité par passage sur un lit de particules de rafles de maïs servant de support à l'invertase, en vue de l'obtention d'un sirop inverti.

Selon une autre forme de l'invention, un jus de saccharose est traité par passage sur un lit de particules de rafles de maïs servant de support à la lévane saccharase: on obtient un jus contenant du polyfructose et du glucose.

Les exemples, ci-après, permettront de mieux comprendre l'invention:

L'invertase est une enzyme qui catalyse l'hydrolyse de saccharose en glucose et fructose, dont on a, selon l'invention, réalisé la fixation sur des supports solides insolubles dans l'eau, supports contenant à la fois de la cellulose et de la lignine.

Plus particulièrement on a étudié la fixation de l'invertase sur la partie dure des rafles de maïs dont la teneur en lignine est supérieure à 25%. Cette partie dure a été broyée de façon à obtenir des particules dont les diamètres sont compris entre 170 μ et 280 μ. Leur densité apparente est de 0,4 kg·l⁻¹ à l'état sec et de 1,8 kg·l⁻¹ à l'état mouillé. Leur surface spécifique est de 1 m²·g⁻¹.

L'activité de la préparation enzymatique est déterminée en mettant en contact, pendant 4 min à 40°C, 10 ml de solution de saccharose 0,4M dans du tampon acétate 0,1M, pH 4,5 avec 0,1 ml de solution d'invertase (ou de suspension d'invertase immobilisée) dans le même tampon.

La réaction est arrêté en ajoutant 5 ml du milieu à 0,5 ml de solution de soude 2N. La teneur en sucres réducteurs libérés est ensuite déterminée par la méthode du dinitrosalicylate (lecture de l'absorbance à mn). Un étalonnage est effectué à l'aide de solutions équimoléculaires de glucose et de fructose dans le tampon acétate 0,1M (pH 4,5).

Une unité d'activité enzymatique (U) est définie comme étant la quantité d'enzyme qui provoque la libération de 1 g de sucres réducteurs par minute dans les conditions d'essai.

L'activité spécifique de la préparation d'invertase (SIGMA Chem. Co.) est de 93,3 U/mg d'enzyme.

Le procédé de traitement des particules de rafles de maïs comporte les étapes suivantes:

a) lavage préalable à l'eau distillée pendant 24 h à 25°C pour éliminer les substances solubles, suivi par un séchage à 60°C,
b) oxydation ménagée par mise en contact d'un échantillon de 100 mg avec 20 ml de solution de métaperiodate de sodium dans l'eau distillée à 25°C, dans l'obscurité,
c) amination par réaction des rafles oxydées avec 20 ml de solution de diamine dans le méthanol à 25°C.

On a, pour cette étape, testé les diamines suivantes:
— l'éthylènediamine,
— l'hexaméthylènediamine,
— l'octaméthylènediamine,
— le diamino dicyclohexyl méthane,
— le diamino diphényl méthane,

d) réduction par mise en contact à 25°C soit avec 25 ml de solution de borohydrure de sodium, dans du tampon carbonate 0,05M, pH 10,5, ou dans du méthanol, soit par 20 ml de solution de cyanoborohydrure de sodium, dans du tampon phosphate 0,05M, pH 6,5,
e) activation par une solution de glutaraldéhyde dans du tampon pyrophosphate 0,05M, pH 8,6 à 25°C,
f) fixation de l'invertase en solution dans du tampon acétate 0,1M, pH 4,5, à 4°C.

Chaque étape est suivie de deux lavages des rafles par remise en suspension pendant 15 min, dans 20 ml du milieu utilisé au cours de l'étape précédente — ne contenant pas l'espèce réactive — puis par trois lavages par remise en suspension dans le milieu utilisé au cours de l'étape suivante.

Les diverses étapes peuvent être schématisées, dans leur principe, de la façon suivante:

*Oxydation*

$$NaIO_4 \longrightarrow$$

*Amination*

$$H_2N-R-NH_2 \longrightarrow$$

*Réduction*

$$\frac{NaBH_4}{ou} \atop NaBH_3CH \longrightarrow$$

*Activation*

$$OHC-(CH_2)_2-CHO \atop (G) \longrightarrow$$

*Immobilisation*

$$\text{enzyme} \atop (E) \longrightarrow$$

Après greffage de l'enzyme, le support est lavé par 20 ml de solution de NaCl 1M dans l'eau distillée afin d'éliminer l'invertase simplement adsorbée. On procède ensuite à la détermination de l'activité retenue sur l'échantillon de rafles.

On a bien évidemment cherché à optimiser les différentes étapes:

1) Oxydation

a) *Influence de la concentration du periodate de sodium*

Les conditions expérimentales choisies sont les suivantes:

*Tableau 1*

| Etape | Oxydation | Amination | Réduction | Activation | Fixation |
|---|---|---|---|---|---|
| Produit | $NaIO_4$ | $C_2H_8N_2$ | $NaBH_4$ | glutaraldéhyde | invertase |
| Milieu | $H_2O$ | $CH_3OH$ | $CH_3OH$ | T. pyrophosphate | T. acétate |
| pH | | | | 8,6 | 4,5 |
| Concentration | variable | 3M | $10\ g \cdot l^{-1}$ | 1,25% | $0,5\ g \cdot l^{-1}$ |
| Durée (h) | 30 | 72 | 4 | 5 | 10 |

On a pu, en faisant varier la concentration en $NaIO_4$, obtenir les résultats présentés dans le tableau 2.

*Tableau 2*

| Concentration $NaIO_4$(M) | 0 | 0,05 | 0,2 | 0,4 |
|---|---|---|---|---|
| Activité (U/g de rafles) | 0 | 0,28 | 1,28 | 1,33 |

En effectuant la réduction par du cyanoborohydrure de sodium (concentration $10\ g \cdot l^{-1}$, tampon phosphate pH 6,5, durée 2 h) à la place du borohydrure de sodium, toutes autres conditions égales par ailleurs, on obtient les résultats présentés dans le tableau 3:

*Tableau 3*

| Concentration $NaIO_4$(M) | 0 | 0,05 | 0,2 | 0,4 |
|---|---|---|---|---|
| Activité (U/g de rafles) | 0 | 0,53 | 2,20 | 2,18 |

On constate donc que, quelle que soit la méthode de réduction utilisée, le greffage optimal de l'enzyme est obtenu au-delà d'une concentration de métaperiodate de sodium 0,2M.

b) *Influence de la durée*

La concentration en métaperiodate étant fixée à 0,2M et toutes les autres conditions expérimentales étant identiques à celles choisies en a (mis à part le milieu réducteur méthanolique remplacé par un tampon phosphate) on obtient les résultats présentés dans le tableau 4:

*Tableau 4*

| Durée d'oxydation (h) | 0 | 2 | 5 | 8,75 | 13 | 24 | 48 |
|---|---|---|---|---|---|---|---|
| Activité (U/g de rafles) | 0 | 0,42 | 0,55 | 0,77 | 0,97 | 1,49 | 1,53 |

Le temps optimal d'oxydation retenu est de 30 h.

Il est à remarquer que l'oxydation paraît procéder en deux étapes:

— une première étape rapide (0-2 h),

— une seconde étape plus lente (2-24 h).

2) Oxydation

a) *Influence de la nature de la diamine*

La durée d'oxydation par du periodate de sodium 0,2M étant fixée à 30 h, les autres conditions expérimentales étant celles choisies en 1a, on obtient les résultats présentés dans le tableau 5:

*Tableau 5*

| Diamine | $C_2H_8N_2$ | $C_6H_{16}N_2$ | $C_8H_{20}N_2$ | $C_{13}H_{26}N_2$ |
|---|---|---|---|---|
| Activité (U/g de rafles) | 1,28 | 0,61 | 0,43 | 0,40 |

Les activités enzymatiques les plus élevées sont donc obtenues lorsqu'on utilise l'éthylènediamine ($C_2H_8N_2$).

b) *Influence de la concentration de l'éthylène-diamine*

Les conditions expérimentales étant celles de l'exemple 1a (mis à part la durée de l'oxydation ramenée à 13 h et le milieu réducteur méthanolique remplacé par un tampon de carbonate), on obtient les résultats présentés dans le tableau 6:

*Tableau 6*

| Concentration de la diamine (M) | 0 | 0,05 | 0,5 | 1 | 5 | 7,5 |
|---|---|---|---|---|---|---|
| Activité (U/g de rafles) | 0 | 0,50 | 0,72 | 0,97 | 1,24 | 1,23 |

On notera qu'une activité catalytique importante est obtenue avec une concentration relativement faible de diamine (0,05M). On considère cependant que la concentration optimale est 3M.

c) *Influence de la durée*

Les conditions expérimentales sont les suivantes:

*Tableau 7*

| Etape | Oxydation | Amination | Réduction | Activation | Fixation |
|---|---|---|---|---|---|
| Produit | $NaIO_4$ | $C_2H_8N_2$ | $NaBH_3CN$ | glutaraldéhyde | invertase |
| Milieu | $H_2O$ | $CH_3OH$ | T. phos. | T. pyrophosphate | T. acétate |
| pH | | | 6,5 | 8,6 | 4,5 |
| Concentration | 0,2M | 3M | $10 \, g \cdot l^{-1}$ | 1,25% | $0,5 \, g \cdot l^{-1}$ |
| Durée (h) | 30 | variable | 2 | 5 | 10 |

Les résultats sont donnés dans le tableau 8:

*Tableau 8*

| Durée d'amination (h) | 0 | 17 | 24 | 48 | 72 | 79 |
|---|---|---|---|---|---|---|
| Activité (U/g de rafles) | 0 | 0,98 | 1,21 | 1,84 | 2,20 | 2,12 |

Une durée de 72 h a donc été retenue pour l'étape d'amination.

3) *Réduction*

a) *Influence de la nature et de la concentration de l'agent de réduction*

Les conditions expérimentales sont les suivantes:

*Tableau 9*

| Etape | Oxydation | Amination | Réduction | Activation | Fixation |
|---|---|---|---|---|---|
| Produit | $NaIO_4$ | $C_2H_8N_2$ | variable | glutaraldéhyde | invertase |
| Milieu | $H_2O$ | $CH_3OH$ | variable | T. pyrophosphate | T. acétate |

*Tableau 9 (suite)*

| Etape | Oxydation | Amination | Réduction | Activation | Fixation |
|---|---|---|---|---|---|
| pH | | | | 8,6 | 4,5 |
| Concentration | 0,2M | 3M | variable | 1,25% | $0,5 \, g \cdot l^{-1}$ |
| Durée (h) | 30 | 72 | 2 | 5 | 10 |

Les résultats sont donnés dans le tableau 10:

*Tableau 10*

| Agent de réduction | Concentration de l'agent de réduction $(g \cdot l^{-1})$ | | |
|---|---|---|---|
| | 0 | 10 | 20 |
| $NaBH_4$ (tampon carbonate 0,05M) pH 10,5 | 1,11 | 1,29 | 1,28 |
| $NaBH_3CN$ (tampon phosphate 0,05M) pH 6,5 | 1,11 | 2,20 | 2,25 |

Le cyanoborohydrure s'avère donc être l'agent de réduction le plus efficace.

b) *Influence de la durée de réaction*

Les conditions expérimentales sont les suivantes:

*Tableau 11*

| Etape | Oxydation | Amination | Réduction | Activation | Fixation |
|---|---|---|---|---|---|
| Produit | $NaIO_4$ | $C_2H_8N_2$ | $NaBH_3CN$ | glutaraldéhyde | invertase |
| Milieu | $H_2O$ | $CH_3OH$ | T. phosphate | T. pyrophosphate | T. acétate |
| pH | | | 6,5 | 8,6 | 4,5 |
| Concentration | 0,2M | 3M | $10 \, g \cdot l^{-1}$ | 1,25% | $0,5 \, g \cdot l^{-1}$ |
| Durée (h) | 30 | 72 | variable | 5 | 10 |

Les résultats sont donnés dans le tableau 12:

*Tableau 12*

| Temps de réduction (h) | 0 | 2 | 4 |
|---|---|---|---|
| Activité (U/g de rafles) | 1,11 | 2,20 | 2,15 |

4) *Activation*

a) *Influence de la concentration du glutaraldéhyde et de la durée*

Les conditions expérimentales sont identiques à celles de l'essai 3b.

Les résultats, exprimés en U/g de rafles, sont donnés dans le tableau 13.

*Tableau 13*

| Temps d'activation (h) | Concentration du glutaraldéhyde (%, V/V) | | | | |
|---|---|---|---|---|---|
| | 0 | 0,5 | 1,25 | 5 | 10 |
| 0 | — | — | 1,34 | — | — |
| 0,5 | — | — | 1,42 | — | — |
| 3 | — | — | 1,70 | — | — |
| 5 | 1,34 | 2,26 | 2,33 | 2,30 | 2,32 |
| 11 | — | — | 2,34 | — | — |

La concentration optimale est donc de 1,25% et la durée de 5 h.

## 5) Fixation de l'invertase

### a) Influence de la concentration de l'invertase et de la durée de fixation

Les conditions expérimentales sont identiques à celles de l'essai 3b.
Les résultats sont donnés dans le tableau 14.

*Tableau 14*

| Temps de fixation (h) | Concentration de l'invertase $(g \cdot l^{-1})$ | | | | | |
|---|---|---|---|---|---|---|
| | 0,05 | 0,1 | 0,5 | 1 | 2 | 5 |
| 10 | 0,39 | 1,01 | 2,36 | 3,30 | 3,52 | 3,56 |
| 24 | — | — | — | 3,82 | — | — |
| 35 | — | — | — | 4,08 | — | — |

La concentration optimale de l'invertase est donc de 2 $g \cdot l^{-1}$ et la durée optimale de fixation est de 30 h.

Le tableau 15 regroupe les conditions optimales de fixation de l'invertase sur les rafles de maïs.

*Tableau 15*

| Etape | Oxydation | Amination | Réduction | Activation | Fixation |
|---|---|---|---|---|---|
| Produit | $NaIO_4$ | $C_2H_8N_2$ | $NaBH_3CN$ | glutaraldéhyde | invertase |
| Milieu | $H_2O$ | $CH_3OH$ | T. phosphate | T. pyrophosphate | T. acétate |
| pH | | | 6,5 | 8,6 | 4,5 |
| Concentration | 0,2M | 3M | 10 $g \cdot l^{-1}$ | 1,25% | 2 $g \cdot l^{-1}$ |
| Durée (h) | 30 | 72 | 2 | 5 | 30 |

On a testé d'autres méthodes d'immobilisation de l'invertase sur les rafles de maïs:
— absorption,
— absorption suivie d'une réticulation par le glutaraldéhyde,
— fixation directe sur les rafles oxydées,
— greffage des diamines aromatiques activées par diazotation,
— oxydation des aldéhydes en acides, suivie d'une activation selon la méthode à l'azoture.

Aucune de ces méthodes n'a donné de résultats comparables à ceux obtenus par le procédé selon l'invention.

On a en particulier mis en application la méthode décrite dans le brevet français No 2247472.

Deux supports ont été utilisés:
1) échantillon de rafles entières, broyées mécaniquement et tamisées afin de ne conserver que la fraction de diamètre de particules compris entre 100 et 200 µ,
2) échantillon de Grits d'un diamètre compris entre 170 et 280 µ.

L'immobilisation de l'invertase a été réalisée par mise en contact de 100 mg de support activé avec 20 ml de solution d'invertase (SIGMA Chem. Co. I 5875) à 2 $g \cdot l^{-1}$ dans du tampon acétate 0,1 M, pH 4,5, pendant 30 h sous agitation rotative à 4°C.

Après élimination exhaustive de l'enzyme non fixée, par lavage des supports à l'aide de tampon acétate 0,1 M, pH 4,5, l'activité retenue a été déterminée en mesurant la quantité de sucres réducteurs (glucose et fructose) libérés par action de l'enzyme sur une solution de saccharose 0,4M à 40°C.

Les résultats sont donnés dans le tableau 16:

*Tableau 16*

| Méthode d'immobilisation | Support | Activité enzymatique |
|---|---|---|
| Brevet FR No 2247472 | Rafles entières | 0,049 0,0505 |
| | Grits | 0,036 0,015 |
| Selon la présente invention | Rafles entières | 2,12 2,10 |
| | Grits | 3,10 2,94 |

Les activités enzymatiques obtenues avec des supports traités selon l'invention sont de 40 à 80 fois supérieures à celles obtenues avec les mêmes supports traités selon le brevet français No 2247472.

On a testé également les possibilités d'utilisation en continu de l'invertase immobilisée sur rafles de maïs, dans un réacteur à lit fixe de 10 ml de volume,

8

garni de 1,9 g de rafles portant l'enzyme. Ce réacteur, placé dans une enceinte thermostatée à 40°C, est alimenté en continu à l'aide de solutions de saccharose de concentration variable dans du tampon acétate 0,1 M, pH 4,5.

Les résultats sont donnés dans le tableau 17.

*Tableau 17*

| Concentration initiale de saccharose | Débit d'alimentation (l·h⁻¹) | | | | | | |
|---|---|---|---|---|---|---|---|
| | 0,15 | 0,40 | 0,68 | 0,82 | 0,97 | 1,05 | 1,2 |
| 0,1 M (34,2 g·l⁻¹) (%) | 100 | 100 | 100 | 100 | 100 | 100 | 100 |
| 1 M (342 g·l⁻¹) (%) | 97 | 96 | 80 | 73 | 62 | 57 | 53 |

On atteint donc des productivités horaires maximales qui sont:
— pour une concentration initiale de saccharose 0,1 M: 22 g de saccharose hydrolysés par heure et par gramme de rafles,
— pour une concentration initiale de saccharose 1 M: 117 g de saccharose hydrolysés par heure et par gramme de rafles.

En vue d'étudier la transposition à l'échelle industrielle du procédé d'hydrolyse en continu de jus de saccharose, on a réalisé les expériences suivantes:

Deux réacteurs ont été utilisés:
— réacteur A: volume total: 100 ml
            quantité de rafles: 44,1 g,
— réacteur B: volume total: 1 l
            quantité de rafles: environ 400 g.

Une préparation commerciale d'invertase (SIGMA Chem. Co.) a été immobilisée sur des rafles de maïs dont le diamètre moyen était de 0,81 mm. Les résultats obtenus pour les 2 réacteurs successivement préparés sont regroupés dans le tableau 18:

*Tableau 18*

| | Activité[a] (U) | Rendement de l'immobilisation[b] (%) |
|---|---|---|
| Réacteur A | 0,32 | 3 |
| Réacteur B | 0,84 | 16 |
| Conditions optimales[c] | 1,10 | 19 |

[a] L'activité est exprimée en grammes de sucres réducteurs libérés par mm et par gramme de support dans les conditions d'essai.

[b] Le rendement de l'immobilisation est exprimée comme le rapport de l'activité par milligramme d'enzyme immobilisée à l'activité par mg d'enzyme libre.

[c] Les conditions optimales d'immobilisation ont été déterminées précédemment, au niveau d'échantillons de 100 mg de rafles.

On notera que le problème de l'extrapolation de l'immobilisation de l'invertase de l'échelle d'échantillons de supports de 100 mg à l'échelle de quantités de rafles allant jusqu'à 1 kg a été résolu puisque les résultats obtenus pour le réacteur B sont voisins de ceux obtenus dans les conditions optimales.

Le tableau 19 regroupe les résultats concernant la production de glucose et fructose (exprimés en grammes de sucres réducteurs produits par heure et par gramme de rafles).

La productivité maximale a été déterminée respectivement pour un taux de conversion de 90% et de 100% de saccharose initial.

*(Tableau en page suivante)*

L'extrapolation des résultats du réacteur de 100 ml à celui de 1 l est sensiblement linéaire, les productivités maximales étant dans le même rapport que les activités enzymatiques par gramme de support (0,32 U et 0,84 U respectivement).

L'invertase immobilisée s'avère extrêmement efficace puisque à partir des résultats obtenus avec le réacteur de 1 l pour une solution à 68,4% de saccharose, on obtient, à 50°C, une productivité de 11 g de sucres réducteurs par jour pour le réacteur de 1 l de volume (le taux de conversion étant de 90%).

La stabilité du catalyseur est très bonne car aucune diminution notable d'activité n'a pu être notée après 20 d de fonctionnement continu (avec séjour à 40, 50, 55 et 60°C) pour le réacteur de 1 l.

Le traitement d'un sirop de refonte de sucre pur ou de sirop industriel provenant de la betterave ou de la canne à sucre, titrant environ 70 Brix à une température comprise entre 40 et 60°C, s'est avéré positif.

Toutefois la productivité maximale est alors 2 fois plus faible que pour une solution à environ 70% de saccharose.

Il est néanmoins possible d'envisager l'hydrolyse continue de solutions aussi concentrées de saccharose, moyennant un préchauffage du sirop destiné à en abaisser la viscosité.

Il est important de souligner que les dérivés enzymatiques obtenus selon l'invention permettent de traiter en continu des jus très concentrés de saccharose sans pour autant voir leur activité diminuer.

On comprend aisément l'avantage considérable que l'on en tire sur le plan industriel; volume réduit

*Tableau 19*

| Volume réacteur (l) | Concentration saccharose | Température (°C) | Productivité maximale (g/h × g de support) | |
|---|---|---|---|---|
| | | | à 90% de conversion | à 100% de conversion |
| 0,1 | 1M (342 g/l) | 40 | 3,55 | 2,32 |
| | 2M (684 g/l) | 40 | 2,90 | 2,10 |
| | | 50 | 4,10 | 2,40 |
| | | 55 | 5,30 | 3,30 |
| | | 60 | 5,00 | 2,50 |
| 1 | 1M | 40 | 9,65 | 5,25 |
| | 2M | 40 | 6,50 | 4,90 |
| | | 50 | 11,00 | 6,50 |
| | | 55 | 7,90 | 6,50 |
| | Solution 70 Brix (Akg/l) | 50 | 5,40 | 1,90 |

des installations, économies d'énergie, durée de vie du support enzymatique.

## Revendications

1. Procédé d'obtention de dérivés enzymatiques dans lesquels l'enzyme est fixée par une liaison covalente sur un support insoluble dans l'eau, comportant les étapes suivantes:
a) lavage à l'eau d'un support contenant essentiellement de la cellulose et de la lignine,
b) oxydation ménagée de la fraction osidique du support,
c) condensation d'une diamine sur les groupes aldéhydiques du support oxydé,
d) stabilisation de la liaison amine-support par réduction,
caractérisé en ce que
e) on active les groupes aminés par un dialdéhyde, et en ce que
f) on couple ensuite l'enzyme sur le support activé.

2. Procédé selon la revendication 1, caractérisé en ce que le dialdéhyde est le glutaraldéhyde.

3. Procédé selon la revendication 1, caractérisé en ce que l'enzyme est l'invertase.

4. Procédé selon l'une des revendications 2 ou 3, caractérisé en ce que:
a) le support est lavé à l'eau,
b) l'oxydation est réalisée en milieu aqueux par du périodate de sodium en concentration 0,2M pendant 30 h,

c) l'amination est conduite en milieu méthanol avec de l'éthylènediamine 3M pendant 72 h,
d) la réduction est faite à pH 6,5 en milieu tampon phosphate pendant 2 h à l'aide de cyanoborohydrure dont la concentration est de 10 g·l⁻¹,
e) l'activation des groupes aminés est réalisée à l'aide de glutaraldéhyde à une concentration de 1,25% en milieu tampon pyrophosphate 0,05M pendant 5 h,
f) on met le support activé en contact avec une solution d'invertase à 2 g·l⁻¹ dans un tampon acétate 0,1M pendant 30 h.
Les étapes a, b, c, d, e sont conduites à 25°C et l'étape f à 4°C.

5. Procédé selon la revendication 1, caractérisé en ce que l'enzyme est la lévane saccharase, la lactase ou la dextrane saccharase.

6. Dérivé enzymatique dont l'enzyme est fixée de façon covalente sur un support insoluble dans l'eau, contenant essentiellement de la cellulose et de la lignine, caractérisé en ce qu'il est obtenu selon le procédé de la revendication 1.

7. Dérivé enzymatique selon la revendication 6, caractérisé en ce que l'enzyme est l'invertase.

8. Dérivé enzymatique selon la revendication 6, caractérisé en ce que l'enzyme est la lévane saccharase, la lactase ou la dextrane saccharase.

9. Procédé de traitement de jus sucré, caractérisé en ce que l'on fait passer le jus sur un lit de particules contenant essentiellement de la lignine et de la cellulose sur lesquelles est fixée de façon covalente une enzyme selon l'une des revendications 6 à 8.

10. Procédé selon la revendication 9, caractérisé en ce que le jus sucré contient essentiellement du saccharose.

11. Procédé selon la revendication 10, caractérisé en ce que l'enzyme est l'invertase.

12. Procédé selon l'une des revendications 9 à 11, caractérisé en ce que le jus sucré titre environ 70 Brix.

13. Procédé selon la revendication 12, caractérisé en ce que la température du jus sucré est maintenue entre 40 et 60°C.

## Claims

1. Process for obtaining enzymatic derivatives in which the enzyme is fixed by a covalent bond onto a water-insoluble carrier, which process comprises the following stages:

(a) washing a carrier, essentially containing cellulose and lignin, with water,

(b) controlled oxidation of the oside fraction of the carrier,

(c) condensation of a diamine with the aldehyde groups of the oxidised carrier,

(d) stabilisation of the amine-carrier bond by reduction,

characterised in that

(e) the amine groups are activated by means of a dialdehyde,

and in that

(f) thereafter the enzyme is coupled onto the activated carrier.

2. Process according to claim 1, characterised in that the dialdehyde is glutaraldehyde.

3. Process according to claim 1, characterised in that the enzyme is invertase.

4. Process according to claim 2 or 3, characterised in that

(a) the carrier is washed with water,

(b) the oxidation is carried out in an aqueous medium by means of sodium periodate, at a concentration of 0.2M, for 30 h,

(c) the amination is carried out in a methanol medium with 3M ethylenediamine for 72 h,

(d) the reduction is carried out at pH 6.5 in a phosphate buffer medium for 2 h, using a cyanoborohydride whose concentration is $10 \, g \cdot l^{-1}$,

(e) the activation of the amine groups is effected by means of glutaraldehyde at a concentration of 1.25% in a 0.05M pyrophosphate buffer medium for 5 h,

(f) the activated carrier is brought into contact with an invertase solution of strength $2 \, gl^{-1}$ in a 0.1 M acetate buffer for 30 h,

stages a, b, c, d and e being carried out at 25°C and stage f at 4°C.

5. Process according to claim 1, characterised in that the enzyme is levan-saccharase, lactase or dextran-saccharase.

6. Enzymatic derivative in which the enzyme is fixed covalently to a water-insoluble carrier, essentially containing cellulose and lignin, characterised in that it is obtained in accordance with the process of claim 1.

7. Enzymatic derivative according to claim 6, characterised in that the enzyme is invertase.

8. Enzymatic derivative according to claim 6, characterised in that the enzyme is levan-saccharase, lactase or dextran-saccharase.

9. Process for the treatment of sugar juice, characterised in that the juice is passed over a bed of particles, essentially containing lignin and cellulose onto which an enzyme according to one of claims 6 to 8 is fixed covalently.

10. Process according to claim 9, characterised in that the sugar juice essentially contains sucrose.

11. Process according to claim 10, characterised in that the enzyme is invertase.

12. Process according to one of claims 9 to 11, characterised in that the sugar juice is of approximate strength 70 Brix.

13. Process according to claim 12, characterised in that the temperature of the sugar juice is kept at between 40 and 60°C.

## Patentansprüche

1. Verfahren zur Erlangung von enzymatischen Derivaten, in denen das Enzym durch eine kovalente Verbindung auf einem wasserunlöslichen Träger fixiert ist, bestehend aus den folgenden Schritten:

a) Waschen eines im wesentlichen Cellulose und Lignin enthaltenden Trägers mit Wasser,

b) Oxidieren der osidischen Fraktion des Trägers,

c) Kondensieren eines diamins auf den Aldehydgruppen des oxidierten Trägers,

d) Stabilisieren der Verbindung zwischen Amin und Träger durch Reduktion,

dadurch gekennzeichnet, dass man

e) die aminierten Gruppen durch einen Dialdehyd aktiviert,

und danach

f) das Enzym auf dem aktivierten Träger bindet.

2. Verfahren nach Anspruch 1, dadurch gekennzeichnet, dass der Dialdehyd Glutaraldehyd ist.

3. Verfahren nach Anspruch 1, dadurch gekennzeichnet, dass das Enzym Invertase ist.

4. Verfahren nach einem der beiden Ansprüche 2 oder 3, dadurch gekennzeichnet, dass

a) der Träger mit Wasser gewaschen wird,

b) die Oxidation durch ein Periodat des Natriums in einer Konzentration von 0,2M während 30 h in einem wässerigen Medium durchgeführt wird,

c) die Aminierung in einem Methanolmedium mit Ethylendiamin 3M während 72 h durchgeführt wird,

d) die Reduktion bei einem pH-Wert von 6,5 in einem Phosphatbauschmedium während 2 h mit Hilfe von Cyanborhydrid mit einer Konzentration von $10 \, g \cdot l^{-1}$ durchgeführt wird,

e) die Aktivierung der aminierten Gruppen mit Hilfe von Glutaraldehyd bei einer Konzentration von 1,25% in einem Pyrophosphatbauschmedium von 0,05N während 5 h durchgeführt wird,

f) der aktivierte Träger mit einer Invertaselösung bei 2 g · l⁻¹ in einem Acetatbausch von 0,1 M für 30 h in Berührung gebracht wird,

und die Stufen a, b, c, d, e bei 25°C und die Stufe f bei 4°C durchgeführt wird.

5. Verfahren nach Anspruch 1, dadurch gekennzeichnet, dass das Enzym Levansaccharase, Lactase oder Dextransaccharase ist.

6. Enzymatischer Derivat, dessen Enzym kovalent auf einem im wesentlichen Cellulose und Lignin enthaltenden wasserunlöslichen Träger fixiert ist, dadurch gekennzeichnet, dass er gemäss dem Verfahren nach Anspruch 1 erhalten wird.

7. Enzymatischer Derivat nach Anspruch 6, dadurch gekennzeichnet, dass das Enzym Invertase ist.

8. Enzymatischer Derivat nach Anspruch 6, dadurch gekennzeichnet, dass das Enzym Levansaccharase, Lactase oder Dextransaccharase ist.

9. Verfahren zur Behandlung von gezuckertem Saft, dadurch gekennzeichnet, dass man den Saft über ein Bett von im wesentlichen Lignin und Cellulose enthaltenden Partikeln führt, auf denen ein Enzym nach einem der Ansprüche 6 bis 8 kovalent fixiert ist.

10. Verfahren nach Anspruch 9, dadurch gekennzeichnet, dass der gezuckerte Saft im wesentlichen Saccharose enthält.

11. Verfahren nach Anspruch 10, dadurch gekennzeichnet, dass das Enzym Invertase ist.

12. Verfahren nach einem der Ansprüche 9 bis 11, dadurch gekennzeichnet, dass der gezuckerte Saft bei etwa 70 Brix titriert.

13. Verfahren nach Anspruch 12, dadurch gekennzeichnet, dass die Temperatur des gezuckerten Saftes zwischen 40 und 60°C gehalten wird.